# EUROPÄISCHE PATENTSCHRIFT

(11) **EP 2 136 776 B1**
(45) Veröffentlichungstag und Bekanntmachung des Hinweises auf die Patenterteilung: **26.10.2011**
(21) Anmeldenummer: 08748952.2
(22) Anmeldetag: 17.04.2008
(51) Int. Cl.: A61K 8/37, C11C 3/04

(54) **ESTER VON HEXYLDECANOL MIT KURZKETTIGEN FETTSÄUREN**
ESTER OF HEXYLDECANOL HAVING SHORT-CHAINED FATTY ACIDS
ESTER D'HEXYLDÉCANOL COMPRENANT DES ACIDES GRAS À CHAINE COURTE

(30) Priorität: 26.04.2007 EP 07008476
(43) Veröffentlichungstag der Anmeldung: 30.12.2009
(73) Patentinhaber: Cognis IP Management GmbH, 40589 Düsseldorf (DE)
(72) Erfinder: ANSMANN, Achim, 40699 Erkrath (DE); DIERKER, Markus, 40593 Düsseldorf (DE)
(86) Internationale Anmeldenummer: PCT/EP2008/003067
(87) Internationale Veröffentlichungsnummer: WO 2008/131863

(56) Entgegenhaltungen:
- JP-A- 10 279 527
- US-A- 5 656 664
- PINOL R ET AL: "STRUCTURE-ACTIVITY STUDIES OF FERROELECTRIC AND ANTIFERROELECTRIC IMINE LIGANDS AND THEIR SQUARE-PLANAR COMPLEXES" LIQUID CRYSTALS, TAYLOR AND FRANCIS, ABINGDON, GB, Bd. 31, Nr. 9, September 2004 (2004-09), Seiten 1293-1303, XP001200376 ISSN: 0267-8292
- KIRCHNER G ET AL: "RESOLUTION OF RACEMIC MIXTURES VIA LIPASE CATALYSIS IN ORGANIC SOLVENTS" JOURNAL OF THE AMERICAN CHEMICAL SOCIETY, AMERICAN CHEMICAL SOCIETY, WASHINGTON, DC, US, Bd. 107, Nr. 24, 1985, Seiten 7072-7076, XP002033390 ISSN: 0002-7863

## Beschreibung

### Gebiet der Erfindung

Die Erfindung betrifft Ester von Hexyldodecanol mit kurzkettigen Fettsäuren und ihre Verwendung in kosmetischen und/oder pharmazeutischen Zubereitungen.

### Stand der Technik

Im Bereich kosmetischer Zubereitungen für die Haut- und Haarpflege sowie im Bereich pharmazeutischer Zubereitungen werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper entwickelt und getestet.

Ester von Hexyldecanol mit Fettsäuren sind beispielsweise aus US 6,391,287 (L Oréal) bekannt, dort werden Hexyldecyl carpylate (=Octanoate), Hexyldecyl laurate (=Dodecanoate), Hexyldecyl palmitat (=Hexadecanoate) und Hexyldecyl stearate (0Octadecanoate) als Co-solubilisatoren für bizyklische aromatische Verbindungen beschrieben.

Weiterhin wird die Verbindung Hexyldecyl 2-ethylhexanoate von der Fa. Kokyu Alcohol Co, Ltd unter dem Handelsnamen ICEH vertrieben.

US 5,656,664 beschreibt Ester von alpha-Methylverzweigten Alkoholen mit Säuren und Ihre Verwendung als "conditioning agents" für die Haut. Nachteilig an diesen Estern ist ihre unbefriedigende Sensorik sowie ihre geringe Stabilität, insbesondere bei Temperaturbelastung.

G. Kirchner et al beschreiben im Journal of the American Society., Bd. 107, Nr. 24 von 1985 auf S. 7073 in Schema 11 als Beispiel 11 einen Ester aus 2-Hexadecanol und Butansäure.

Es besteht weiterhin der Bedarf nach neuen Ölkörpem, die über ein flexibleres Einsatzspektrum verfügen (z.B. Kompatibilität mit weiteren kosmetischen Inhaltsstoffen) sowie den hohen Anforderungen an die Sensorik genügen. Von besonderem Interesse sind Ölkörper, welche auf der Haut eine als "leicht" bezeichnetes Gefühl erzeugen. Von großer Bedeutung sind die Verteilbarkeit und Spreitfähigkeit auf der Haut. Der moderne Verbraucher fordert von Ölkörper und den daraus hergestellten Zubereitungen, dass sie ein weiches Hautgefühl erzeugen und dass sich die Haut gepflegt anfühlt. Eine weitere Anforderung an moderne Rohstoffe für kosmetische und/oder pharmazeutische Zubereitungen ist, dass sie auf Basis nachwachsender, pflanzlicher Rohstoffe gewonnen werden können. Von besonderem interesse sind Verbindungen, die sich durch eine vorteilhafte Sensorik auszeichnen, weiterhin von besonderem Interesse sind Verbindungen die lagerstabil sind, insbesondere lagerstabil bei erhöhten Temperaturen.

Aufgabe der vorliegenden Erfindung war es neue Stoffe bereit zu stellen, welche sich für kosmetischen und/oder pharmazeutischen Zubereitungen, insbesondere als Ölkörper/Emollients eignen und ein sensorisch besonders "leichtes" Hautgefühl erzeugen. Weiterhin sollten diese Stoffe auf Basis nachwachsender pflanzlicher Rohstoffe erhältlich sein.

### Gegenstand der Erfindung

Überraschenderweise wurde gefunden, dass Ester von 2-Hexyldecanol mit kurzkettigen Fettsäuren sich eignen für kosmetische und/oder pharmazeutische Zubereitungen, und in diesen Zubereitungen insbesondere als sensorisch besonders vorteilhafte Ölkörper eingesetzt werden können.

Die erfindungsgemäßen Ester zeichnen sich durch eine vorteilhafte Sensorik und durch eine erhöhte Stabilität gegenüber dem im Stand der Technik bekannten Verbindungen aus.

Gegenstand der Erfindung ist die Verwendung von einem oder mehreren Ester von 2-Hexyldecanol mit Fettsäuren der allgemeinen Formel (I)

R₁-COOH,

wobei R₁ einen linearen oder verzweigten, gesättigten oder ungesättigten Alkylrest mit 1 bis 6 C Atomen darstellt, in und/oder zur Herstellung von kosmetischen und/oder pharmazeutischen Zubereitungen.

Die erfindungsgemäßen Ester eignen sich insbesondere als Ölkörper in kosmetischen und/oder pharmazeutischen Zubereitungen.

Gegenstand der Erfindung sind weiterhin Ester von 2-Hexyldecanol mit Fettsäuren der allgemeinen Formel (I)

R₁-COOH,

wobei R₁ einen linearen oder verzweigten, gesättigten oder ungesättigten Alkylrest mit 1 bis 6 C Atomen darstellt, ausgenommen (*R*)-2-Hexyldecyl-butansäure.

Als Alkoholkomponenten des Esters wird 2-Hexyldecanol eingesetzt. 2-Hexyldecanol (synonym 2-Hexyl-1-decanol oder 2-Hexyldecylalcohol) ist kommerziell erhältlich, so z.B. unter dem Handelsnamen Eutanol®G 16 (Fa. Cognis Deutschland GmbH & Co. KG) oder als Exxal®16 (Exxon Chemical Company). 2-Hexyldecanol ist ein so genannter Guerbet-Alkohol, der durch Selbstkondensation von (primären) Alkoholen unter dem Einfluss von Natrium oder Kupfer bei erhöhter Temperatur und Druck erhältlich ist. 2-Hexyldecanol besitzt ein chirales C-Atom und kann somit als R sowie als S Enantiomer vorliegen, als beliebige Mischung der zwei Enantiomere, sowie als Racemat. Ester von 2-Hexyldecanol im Sinne dieser Erfindung umfassen sowohl die Ester der jeweiligen Enantiomere, Mischungen dieser Enantiomere, als auch die entsprechenden Racemate. Kommerziell erhältliches 2-Hexyldecanol ist in der Regel das Racemat.

Als Fettsäurekomponente des Esters werden Fettsäuren der allgemeinen Formel (I)

R₁-COOH eingesetzt

wobei R₁ einen linearen oder verzweigten, gesättigten oder ungesättigten Alkylrest mit 1 bis 6 C Atomen darstellt.

Als R₁ sind beispielsweise geeignet: Methyl, Ethyl, Propyl-, iso-Propyl [=1-Methylethyl-], Propenyl-, Isobutyl [=2-Methylpropyl], sec-Butyl [=1-Methylpropyl], tert-Butyl [=1,1-Dimethylethyl], But-2-enyl, But-3-enyl, But-1-enyl, n-Pentyl, 1-Methylbutyl-, 2-Methylbutyl-, 3-Methylbutyl, 1-Ethylpropyl, 1,1-Dimethylpropyl, 1,2-Dimethylpropyl, 2,2-Dimethylpropyl, 1-Pentenyl-, 2-Pentenyl-, 3-Pentenyl-, 4-Pentenyl, Hexyl-, 1-Methylpentyl-, 2-Methylpentyl, 3-Methylpentyl, 4-Methylpentyl, 1-Ethylbutyl-, 2-Ethylbutyl-, 3-Ethylbutyl-,1-Hexenyl, 2-Hexenyl, 3-Hexenyl, 4-Hexenyl-, 5-Hexenyl.

In einer bevorzugten Ausführungsform der Erfindung wird eine Fettsäure der allgemeinen Formel (I) eingesetzt, in der R₁ einen linearen oder verzweigten, gesättigten Alkylrest mit 1 bis 6 C Atomen darstellt, besonders bevorzugt einen linearen, gesättigten Alkylrest mit 1 bis 6 C Atomen.

Besonders geeignete Ester im Sinne der Erfindung sind
2-Hexyldecyl-essigsäureester (R₁ = CH₃),
2-Hexyldecyl-propansäureester (R₁ = C₂H₅),
2-Hexyldecyl-pentansäureester (R₁ = C₄H₉),
2-Hexyldecyl-hexansäureester (R₁ C₅H₁₁),
2-Hexyldecyl-heptansäureester (R₁ = C₆H₁₃),

Besonders vorteilhaft im Sinne der Erfindung zur verwendende Ester sind
2-Hexyldecyl-essigsäureester (R₁ = CH₃),
2-Hexyldecyl-propansäureester (R₁ = C₂H₅),
2-Hexyldecyl-butansäureester (R₁ = C₃H₇),
2-Hexyldecyl-pentansäureester (R₁ C₄H₉),
2-Hexyldecyl-hexansäureester (R₁ C₅H₁₁),
2-Hexyldecyl-heptansäureester (R₁ = C₆H₁₃),

### Herstellung

Die Herstellung der erfindungsgemäßen Ester erfolgt nach dem Fachmann bekannten Methoden der Veresterung. So kann z.B. die Fettsäure zusammen mit dem Hexyldecanol in Gegenwart eines Katalysators verestert werden.

Sowohl die Alkoholkomponenten als auch die Fettsäurekomponente können aus pflanzlichen Rohstoffen, wie beispielsweise Palmkern- oder Kokosöl gewonnen werden. Somit sind die erfindungsgemäßen Ester vollständig auf Basis nachwachsender Rohstoffe erhältlich.

### Verwendung in kosmetischen und/oder pharmazeutischen Zubereitungen

Überraschenderweise wurde festgestellt, dass sich die erfindungsgemäßen Ester besonders eignen zur Herstellung von kosmetischen Zubereitungen, sie eignen sich insbesondere als Ölkörper/Emollients und/oder Konsistenzgeber in kosmetischen Zubereitungen. Die erfindungsgemäßen Ester eignen sich weiterhin zur Herstellung von pharmazeutischen Zubereitungen, wobei die erfindungsgemäßen als technische Hilfsstoffe, wie z.B. Ölkörper eingesetzt werden. Die erfindungsgemäßen Ester können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Bevorzugt ist die Verwendung der erfindungsgemäßen Ester als Ölkörper.

Die erfindungsgemäßen Ester können in kosmetischen Formulierungen als sog. ,light emollients' verwenden werden, um spezielle Eigenschaften, wie z.B. Spreitverhalten oder Flüchtigkeit einzustellen. Die erfindungsgemäßen Ester ermöglichen weiterhin viskositätsstabile kosmetische Formulierungen herzustellen. Die erfindungsgemäßen Ester zeichnen sich gegenüber den 1-Methylverzweigten Estern des Standes der Technik durch eine verbesserte Sensorik sowie durch eine erhöhte Stabilität, insbesondere bei Temperaturbelastung aus.

Die erfindungsgemäßen Ester können sowohl einzeln als auch in beliebigen Mischungen untereinander eingesetzt werden.

### Beispiele

### Herstellungsbeispiel:

1 mol Hexansäure (116 g) Hexansäure,1,1 mol (297 g) 2-Hexyldecanol (Guerbitol ®16) 16 sowie 0,22g Fascat® 2001 (Zinnoxalat) wurden zusammen gegeben und für 3h auf 240°C am Wasserabscheider erhitzt, Anschließend wurde das Produkt über eine 30 cm Kolonne destilliert (153-168 °C bei 0,8 mbar). Das Produkt fällt als farbloses und geruchloses Öl an.

## Patentansprüche

1. Verwendung von einem oder mehreren Ester von 2-Hexyldecanol mit Fettsäuren der allgemeinen Formel (I)
R₁-COOH,
wobei R₁ einen linearen oder verzweigten, gesättigten oder ungesättigten Alkylrest mit 1 bis 6 C Atomen darstellt, in und/oder zur Herstellung von kosmetischen und/oder pharmazeutischen Zubereitungen.

2. Verwendung nach Anspruch 1 als Ölkörper.

3. Ester von 2-Hexyldecanol mit Fettsäuren der allgemeinen Formel (I)
R₁-COOH,
wobei R₁ einen linearen oder verzweigten, gesättigten Alkylrest mit 1 bis 6 C Atomen darstellt, ausgenommen (*R*)-2-Hexyldecyl-butansäure, oder R₁ ausgewählt ist aus der gruppe bestehend aus Propenyl-, But-2-enyl, But-3-enyl, But-1-enyl, Pentenyl, 1-Hexenyl. 2-Hexenyl, 3-Hexenyl, 4-Hexenyl-, 5-Hexenyl.

4. Ester nach Anspruch 3 ausgewählt aus der Gruppe bestehend aus 2-Hexyldecyl-essigsäureester, 2-Hexyldecyl-propansäureester, 2-Hexyldecyl-pentansäureester, 2-Hexyldecyl-hexansäureester, und 2-Hexyldecyl-heptansäureester.

## Claims

1. Use of one or more esters of 2-hexyldecanol with fatty acids of the general formula (I)
R₁-COOH,
where R₁ is a linear or branched, saturated or unsaturated alkyl radical having 1 to 6 carbon atoms, in and/or for the production of cosmetic and/or pharmaceutical preparations.

2. Use according to Claim 1 as oil body.

3. Ester of 2-hexyldecanol with fatty acids of the general formula (I)
R₁-COOH,
where R₁ is a linear or branched, saturated alkyl radical having 1 to 6 carbon atoms, with the exception of (R)-2-hexyldecylbutanoic acid, or R₁ is selected from the group consisting of propenyl, but-2-enyl, but-3-enyl, but-1-enyl, 1-pentenyl, 2-pentenyl, 3-pentenyl, 4-pentenyl, 1-hexenyl, 2-hexenyl, 3-hexenyl, 4-hexenyl, 5-hexenyl.

4. Ester according to Claim 3, selected from the group consisting of 2-hexyldecylacetic acid ester, 2-hexyldecylpropanoic acid ester, 2-hexyldecylpentanoic acid ester, 2-hexyldecylhexanoic acid ester and 2-hexyldecylheptanoic acid ester.

## Revendications

1. Utilisation d'un ou plusieurs esters du 2-hexyldécanol et d'acides gras de formule générale (I)
R₁-COOH,
dans laquelle R₁ est un radical alkyle à chaîne droite ou ramifiée, saturé ou insaturé, ayant 1 à 6 atomes de carbone, lors de la fabrication de préparations cosmétiques et/ou pharmaceutiques, ou pour cette fabrication.

2. Utilisation selon la revendication 1, en tant qu'huile pour le corps.

3. Esters du 2-hexyldécanol et d'acides gras de formule générale (I),
R₁-COOH,
dans laquelle R₁ est un radical alkyle à chaîne droite ou ramifiée, saturé, ayant 1 à 6 atomes de carbone, à l'exception de l'acide (R)-2-hexyldécyl-butanoïque, ou R¹ est choisi dans le groupe consistant en les groupes propényle, but-2-ényle, but-3-ényle, but-1-ényle, 1-pentényle, 2-pentényle, 3-pentényle, 4-pentényle, 1-hexényle, 2-hexényle, 3-hexényle, 4-hexényle, 5-hexényle.

4. Esters selon la revendication 3, choisis dans le groupe consistant en les esters de l'acide 2-hexyldécylacétique, les esters de l'acide 2-hexyldécyl-propanoïque, les esters de l'acide 2-hexyldécyl-pentanoïque, les esters de l'acide 2-hexyldécyl-hexanoïque et les esters de l'acide 2-hexyldécyl-heptanoïque.
